# EUROPEAN PATENT APPLICATION

(11) **EP 0 863 134 A1**
(43) Date of publication of application: **09.09.1998**
(21) Application number: 97302918.4
(22) Date of filing: 29.04.1997
(51) Int. Cl.: C07C 317/24, A61K 31/12

(54) **2-(3,5-difluorophenyl)-3-(4-(methyl-sulfonyl)phenyl)-2-cyclopenten-1-one useful as an inhibitor of cyclooxygenase-2**

(30) Priority: 07.03.1997 US 40049 P; 15.04.1997 GB 9707643
(71) Applicant: MERCK FROSST CANADA INC., Kirkland Quebec H9H 3L1 (CA)
(72) Inventor: Black, Cameron, Kirkland, Quebec H9H 3L1 (CA)
(74) Representative: Cole, William Gwyn

(57) **Abstract**

The invention encompasses the novel compound A, 2-(3,5-difluorophenyl)-3-(4-(methylsulfonyl)phenyl)-2-cyclopenten-1-one, pharmaceutical compositions and use of the compound in the preparation of medicaments for the treatment of cyclooxygenase-2 mediated diseases.

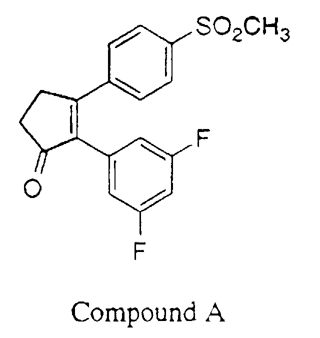

## Description

### BACKGROUND OF THE INVENTION

This invention relates to methods of treating cyclooxygenase mediated diseases and certain pharmaceutical compositions therefor.

Non-steroidal, antiinflammatory drugs exert most of their antiinflammatory, analgesic and antipyretic activity and inhibit hormone-induced uterine contractions and certain types of cancer growth through inhibition of prostaglandin G/H synthase, also known as cyclooxygenase. Initially, only one form of cyclooxygenase was known, this corresponding to cyclooxygenase-1 (COX-1) or the constitutive enzyme, as originally identified in bovine seminal vesicles. More recently the gene for a second inducible form of cyclooxygenase, cyclooxygenase-2 (COX-2) has been cloned, sequenced and characterized initially from chicken, murine and human sources. This enzyme is distinct from the COX-1 which has been cloned, sequenced and characterized from various sources including the sheep, the mouse and man. The second form of cyclooxygenase, COX-2, is rapidly and readily inducible by a number of agents including mitogens, endotoxin, hormones, cytokines and growth factors. As prostaglandins have both physiological and pathological roles, we have concluded that the constitutive enzyme, COX-1, is responsible, in large part, for endogenous basal release of prostaglandins and hence is important in their physiological functions such as the maintenance of gastrointestinal integrity and renal blood flow. In contrast, we have concluded that the inducible form, COX-2, is mainly responsible for the pathological effects of prostaglandins where rapid induction of the enzyme would occur in response to such agents as inflammatory agents, hormones, growth factors, and cytokines. Thus, a selective inhibitor of COX-2 will have similar antiinflammatory, antipyretic and analgesic properties to a conventional non-steroidal antiinflammatory drug, and in addition would inhibit hormone-induced uterine contractions and have potential anti-cancer effects, but will have a diminished ability to induce some of the mechanism-based side effects. In particular, such a compound should have a reduced potential for gastrointestinal toxicity, a reduced potential for renal side effects, a reduced effect on bleeding times and possibly a lessened ability to induce asthma attacks in aspirin-sensitive asthmatic subjects.

Furthermore, such a compound will also inhibit prostanoid-induced smooth muscle contraction by preventing the synthesis of contractile prostanoids and hence may be of use in the treatment of dysmenorrhea, premature labour, asthma and eosinophil related disorders. It will also be of use in the treatment of Alzheimer's disease, for decreasing bone loss particularly in postmenopausal women (i.e. treatment of osteoporosis) and for the treatment of glaucoma.

A brief description of the potential utility of cyclooxygenase-2 inhibitors is given in an article by John Vane, Nature, Vol. 367, pp. 215-216, 1994, and in an article in Drug News and Perspectives, Vol. 7, pp- 501-512, 1994.

### SUMMARY OF THE INVENTION

The invention encompasses the novel compound 2-(3,5-difluorophenyl)-3-(4-(methylsulfonyl)phenyl)-2-cyclopenten-1-one, pharmaceutical compositions and use of the compound in the preparation of medicaments for the treatment of cyclooxygenase-2 mediated diseases.

### DETAILED DESCRIPTION OF THE INVENTION

In one aspect the invention encompasses the compound 2-(3,5-difluorophenyl)-3-(4-(methylsulfonyl)phenyl)-2-cyclopenten-1-one:

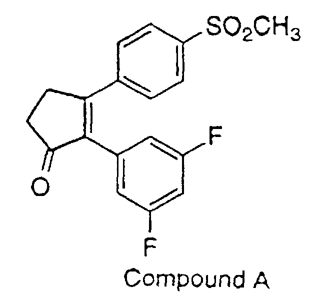

In a second aspect the invention encompasses pharmaceutical compositions useful for the treatment of COX-2 mediated diseases comprising 2-(3,5-difluorophenyl)-3-(4-(methylsulfonyl)phenyl)-2-cyclopenten-1-one and a pharmaceutically acceptable carrier.

In a third aspect the invention encompasses pharmaceutical compositions comprising 2-(3,5-difluorophenyl)-3-(4-(methylsulfonyl)phenyl)-2-cyclopenten-1-one and a pharmaceutically acceptable carrier.

In a fourth aspect the invention encompasses the use 2-(3,5-difluorophenyl)-3-(4-(methylsulfonyl)phenyl)-2-cyclopenten-1-one in the preparation of a medicament for the treatment of COX-2 mediated diseases.

In a further aspect compositions, methods of treatment, and uses involving oral administration is preferred.

In a further aspect, compositions, methods of treatment and use involving once a day or twice a day administration is preferred.

The following abbreviations have the indicated meanings:
- AA: = arachidonic acid
- CHO: = chinese hamster ovary
- CMC: = 1-cyclohexyl-3-(2-morpholinoethyl) carbodiimidemetho-*p*-toluenesulfonate
- COX: = cyclooxygenase
- DMF: = N,N-dimethylformamide
- HBSS: = Hanks balanced salt solution
- HEPES: = N-[2-Hydroxyethyl]piperazine-N¹-[2-ethanesulfonic acid]
- HWB: = human whole blood
- LPS: = lipopolysaccharide
- mCPBA: = meta-chloroperbenzoic acid
- MMPP: = magnesium monoperoxyphthalate
- NSAID: = non-steroidal anti-inflammatory drug
- PDC: = pyridinium dichromate
- r.t.: = room temperature
- THF: = tetrahydrofuran

The Compound A is useful for the relief of pain, fever and inflammation of a variety of conditions including rheumatic fever, symptoms associated with influenza or other viral infections, common cold, low back and neck pain, dysmenorrhea, headache, toothache, sprains and strains, myositis, neuralgia, synovitis, arthritis, including rheumatoid arthritis, degenerative joint diseases (osteoarthritis), gout and ankylosing spondylitis, bursitis, burns, injuries following surgical and dental procedures. In addition, such a compound may inhibit cellular neoplastic transformations and metastic tumour growth and hence can be used in the treatment of cancer, such as cancer of the colon. Compound A may also be of use in the treatment and/or prevention of cyclooxygenase-mediated proliferative disorders such as may occur in diabetic retinopathy and tumour angiogenesis.

Compound A will also inhibit prostanoid-induced smooth muscle contraction by preventing the synthesis of contractile prostanoids and hence may be of use in the treatment of dysmenorrhea, premature labour, asthma and eosinophil related disorders. It will also be of use in the treatment of Alzheimer's disease, for decreasing bone loss particularly in postmenopausal women (i.e. treatment of osteoporosis), and for treatment of glaucoma.

By virtue of its high cyclooxygenase-2 (COX-2) activity and/or its specificity for cyclooxygenase-2 over cyclooxygenase-1 (COX-1), Compound A will prove useful as an alternative to conventional non-steroidal antiinflammatory drugs (NSAID'S) particularly where such non-steroidal antiinflammatory drugs may be contra-indicated such as in patients with peptic ulcers, gastritis, regional enteritis, ulcerative colitis, diverticulitis or with a recurrent history of gastrointestinal lesions; GI bleeding, coagulation disorders including anemia such as hypoprothrombinemia, haemophilia or other bleeding problems; kidney disease; those prior to surgery or taking anticoagulants.

Similarly, Compound A, will be useful as a partial or complete substitute for conventional NSAID'S in preparations wherein NSAIDS are presently co-administered with other agents or ingredients. Thus in further aspects, the invention encompasses pharmaceutical compositions for treating cyclooxygenase-2 mediated diseases as defined above comprising a non-toxic therapeutically effective amount of the compound of Formula I as defined above and one or more ingredients such as another pain reliever including acetominophen or phenacetin; a potentiator including caffeine; an H₂-antagonist, aluminum or magnesium hydroxide, simethicone, a decongestant including phenylephrine, phenylpropanolamine, pseudoephedrine, oxymetazoline, ephinephrine, naphazoline, xylometazoline, propylhexedrine, or levodesoxyephedrine, an antiitussive including codeine, hydrocodone, caramiphen, carbetapentane, or dextramethorphan; a prostaglandin including misoprostol, enprostil, rioprostil, ornoprostol or rosaprostol; a diuretic; a sedating or non-sedating antihistamine. In addition the invention encompasses a method of treating cyclooxygenase mediated diseases comprising: administration to a patient in need of such treatment a non-toxic therapeutically effective amount of the compound A, optionally co-administered with one or more of such ingredients as listed immediately above.

For the treatment of any of these cyclooxygenase mediated diseases Compound A may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, cattle sheep, dogs, cats, etc., the compound of the invention is effective in the treatment of humans.

As indicated above, pharmaceutical compositions for treating cyclooxygenase-2 mediated diseases as defined may optionally include one or more ingredients as listed above.

The pharmaceutical compositions containing the active ingredients may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the technique described in the U.S. Patent 4,256,108; 4,166,452; and 4,265,874 to form osmotic therapeutic tablets for control release.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredients is mixed with water or miscible solvents such as propylene glycol, PEGs and ethanol, or an oil medium, for example peanut oil, liquid paraffin, or olive oil.

Aqueous suspensions contain the active materials in admixture with excipients suitable for the manufacture of aqueous suspensions. Such excipients are suspending agents, for example, sodium carboxymethylcellulose, methylcellulose, hydroxy-propylmethycellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose, saccharin or aspartame.

Oily suspensions may be formulated by suspending the active ingredients in a vegetable oil, for example, arachis oil, olive oil, sesame oil or coconut oil, or in mineral oil such as liquid paraffin. The oily suspensions may contain a thickening agent, for example, beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredients in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The pharmaceutical compositions of the invention may also be in the form of an oil-in-water emulsions. The oily phase may be a vegetable oil, for example, olive oil or arachis oil, or a mineral oil, for example, liquid paraffin or mixtures of these. Suitable emulsifying agents may be naturally-occurring phosphatides, for example, soy bean, lecithin, and esters or partial esters derived from fatty acids and hexitol anhydrides, for example, sorbitan monooleate, and condensation products of the said partial esters with ethylene oxide, for example, polyoxyethylene sorbitan monooleate. The emulsions may also contain sweetening and flavouring agents.

Syrups and elixirs may be formulated with sweetening agents, for example, glycerol, propylene glycol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example, as a solution in 1,3-butane diol. Among the acceptable vehicles and solvents that may be employed are water, Ringer's solution and isotonic sodium chloride solution. Cosolvents such as ethanol, propylene glycol or polyethylene glycols may also be used. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compound A may also be administered in the form of a suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, gels, solutions or suspensions, etc., containing the compound A are employed. (For purposes of this application, topical application shall include mouth washes and gargles.) Topical formulations may generally be comprised of a pharmaceutical carrier, cosolvent, emulsifier, penetration enhancer, preservative system, and emollient.

Other suitable formulations are set forth in U.S. Patent No. 5,474,995. We have found the following oral formulations to be of particular value:

Rapidisc® - In view of the above mentioned characteristics, 2-(3,5-difluorophenyl)-3-(4-(methylsulfonyl)phenyl)-2-cyclopenten-1-one is particularly well suited for a rapid dissolving sublingual formulation. For example, due to the lack of GI side-effects, the agent need not be take with a large amount of water. Suitable Rapidisc® formulations and methods of making same are disclosed in US 4,305,502, US 4,371,516, US 4,470,202, US 4,758,598, US 4,754,597, US 5,046,618 and US 5,188,882, all of which are hereby incorporated by reference.

As mentioned elsewhere in this specification, we have found 2-(3,5-difluorophenyl)-3-(4-(methylsulfonyl)phenyl)-2-cyclopenten-1-one to possess a surprising combination of attributes. Not only is compound A active, potent, safe and effective at modest oral dosages of 10 to 250 mg of agent per day, but in addition Compound A possesses a half-life in humans of sufficient length that one or two oral doses of 10 to 250 mg of active agent per day will provide effective safe anti-inflammatory treatment over a 24 hour period. Such agents are particularly useful in the treatment of chronic indications, such as rheumatoid and osteo arthritis as well as Alzheimer's Disease.

Oral and intravenous dosage levels for agent 2-(3,5-difluorophenyl)-3-(4-(methylsulfonyl)phenyl)-2-cycloponten-1-one are of the order of from about 10 to 250 mg per patient once or twice a day.

The amount of active agent that may be combined with the carrier materials to produce a single dosage form will vary depending upon the host treated and the particular mode of administration. For example, a formulation intended for the oral administration of humans may contain from 10 to 250 mg of agent compounded with an appropriate and convenient amount of carrier material which may vary from about 5 to about 95 percent of the total composition. Dosage unit forms may typically contain 10, 20, 30, 40, 50, 60, 70, 80, 90, 100, 125 or 250 mg of active agent.

It will be understood, however, that the specific dose level for any particular patient will depend upon a variety of factors including the age, body weight, general health, sex, diet, time of administration, rate of excretion, drug combination and the type and severity of the particular disease undergoing therapy. For many patients, a dosage range of 10 to 50 or 60 to 120 mg once or twice a day is preferred.

For long term therapy, such as in the treatment of chronic diseases including rheumatoid arthritis, osteoarthritis or Alzheimer disease, a dosage of 10 to 50 or 60 to 120 mg once or twice day is preferred. More particularly, for the treatment of osteoarthritis, a dosage of 10, 20, 30, 40, 50, 60, 70, 80, 90 or 100 mg once or twice a day is preferred, whereas for the treatment of rheumatoid arthritis, 10, 20, 30, 40, 50, 60, 70, 80, 90 or 100 mg once or twice a day is preferred. For the treatment of non-chronic indications such as headache or post-operative swelling and pain, 10, 20, 30, 40, 50, 60, 70, 80, 90 or 100 mg once or twice a day is preferred.

Accordingly, in one aspect the invention is directed to a unit dose oral form which comprises from 10 to 250 mg of the cyclooxygenase inhibitor, for example, 10 to 50 or 60 to 120 mg.

In another aspect this invention is directed to a pharmaceutical composition for the treatment of cyclooxygenase-2 mediated diseases, said composition suitable for once or twice a day oral administration, said composition comprising a 10 to 250 mg of 2-(3,5-difluorophenyl)-3-(4-(methylsulfonyl)phenyl)-2-cyclopenten-1-one, and a pharmaceutical carried therefor.

Within this aspect there is a first genus of compositions comprising 10 to 50 mg of 2-(3,5-difluorophenyl)-3-(4-(methylsulfonyl)phenyl)-2-cyclopenten-1-one.

Within this aspect there is a second genus of compositions comprising 60 10 120 mg of 2-(3,5-difluorophenyl)-3-(4-(methylsulfonyl)phenyl)-2-cyclopenten-1-one.

The amount of active ingredient that may be combined with the carrier materials to produce a single dosage form will vary depending upon the host treated and the particular mode of administration. For example, a formulation intended for the oral administration of humans may contain from 10 mg to 250 mg of active agent compounded with an appropriate and convenient amount of carrier material which may vary from about 5 to about 95 percent of the total composition. Dosage unit forms will generally contain between from about 10 mg to about 250 mg of an active ingredient, typically 10 mg, 25 mg, 50 mg, 100 mg, 125 mg, or 250 mg.

It will be understood, however, that the specific dose level for any particular patient will depend upon a variety of factors including the age, body weight, general health, sex, diet, time of administration, route of administration, rate of excretion, drug combination and the severity of the particular disease undergoing therapy.

### Assays for determining Biological Activity

The compound A can be tested using the following assays to determine their cyclooxygenase-2 inhibiting activity.

### INHIBITION OF CYCLOOXYGENASE ACTIVITY

### Whole cell assays for COX-2 and COX-1 using CHO transfected cell lines

Chinese hamster ovary (CHO) cell lines which have been stably transfected with an eukaryotic expression vector pCDNAIII containing either the human COX-1 or COX-2 cDNA's are used for the assay. These cell lines are referred to as CHO [hCOX-1] and CHO [hCOX-2], respectively. For cyclooxygenase assays, CHO[hCOX-1] cells from suspension cultures and CHO[hCOX-2] cells prepared by trypsinization of adherent cultures are harvested by centrifugation (300 x g, 10 min) and washed once in HBSS containing 15 mM HEPES, pH 7.4, and resuspended in HBSS, 15 mM HEPES, pH 7.4, at a cell concentration of 1.5 x 10⁶ cells/ml. Drugs to be tested are dissolved in DMSO to 66.7-fold the highest test drug concentration. Compounds are typically tested at 8 concentrations in duplicate using serial 3-fold serial dilutions in DMSO of the highest drug concentration. Cells (0.3 x 10⁶ cells in 200 µl) are preincubated with 3 µl of the test drug or DMSO vehicle for 15 min at 37°C. Working solutions of peroxide-free AA (5.5 µM and 110 µM AA for the CHO [hCOX-1] and CHO [COX-2] assays, respectively) are prepared by a 10-fold dilution of a concentrated AA solution in ethanol into HBSS containing 15 mM HEPES, pH 7.4. Cells are then challenged in the presence or absence of drug with the AA/HBSS solution to yield a final concentration of 0.5 µM AA in the CHO[hCOX-1] assay and a final concentration of 10 µM AA in the CHO[hCOX-2] assay. The reaction is terminated by the addition of 10 µl 1 N HCl followed by neutralization with 20 µl of 0.5 N NaOH. The samples are centrifuged at 300 x g at 4°C for 10 min, and an aliquot of the clarified supernatant is appropriately diluted for the determination of PGE₂ levels using an enzyme-linked immunoassay for PGE₂ (Correlate PGE₂ enzyme immunoassay kit, Assay Designs, Inc.). Cyclooxygenase activity in the absence of test compounds is determined as the difference in PGE₂ levels of cells challenged with arachidonic acid versus the PGE₂ levels in cells mock-challenged with ethanol vehicle. Inhibition of PGE₂ synthesis by test compounds is calculated as a percentage of the activity in the presence of drug versus the activity in the positive control samples.

### Assay of COX-1 Activity from U937 cell microsomes

U 937 cells are pelleted by centrifugation at 500 x g for 5 min and washed once with phosphate-buffered saline and repelleted. Cells are resuspended in homogenization buffer consisting of 0.1 M Tris-HCl, pH 7.4, 10 mM EDTA, 2 µg/ml leupeptin, 2 µg/ml soybean trypsin inhibitor, 2 µg/ml aprotinin and 1 mM phenyl methyl sulfonyl fluoride. The cell suspension is sonicated 4 times for 10 sec and is centrifuged at 10,000 x g for 10 min at 4° C. The supernatant is centrifuged at 100,000 x g for 1 hr at 4° C. The 100,000 x g microsomal pellet is resuspended in 0.1 M Tris-HCl, pH 7.4, 10 mM EDTA to approximately 7 mg protein/ml and stored at -80° C.

Microsomal preparations are thawed immediately prior to use, subjected to a brief sonication, and then diluted to a protein concentration of 125 µg/ml in 0.1 M Tris-HCl buffer, pH 7.4 containing 10 mM EDTA, 0.5 mM phenol, 1 mM reduced glutathione and 1 µM hematin. Assays are performed in duplicate in a final volume of 250 µl. Initially, 5 µl of DMSO vehicle or drug in DMSO are added to 20 µl of 0.1 M Tris-HCl buffer, pH 7.4 containing 10 mM EDTA in wells of a 96-deepwell polypropylene titre plate. 200 µl of the microsomal preparation are then added and pre-incubated for 15 min at room temperature before addition of 25 µl of 1 M arachidonic acid in 0.1 M Tris-HCl and 10 mM EDTA, pH 7.4. Samples are incubated for 40 min at room temperature and the reaction is stopped by the addition of 25 µl of 1 N HCl. Samples are neutralized with 25 µl of 1 N NaOH prior to quantitation of PGE₂ content by radioimmunoassay (Dupont-NEN or Amersham assay kits). Cyclooxygenase activity is defined as the difference between PGE₂ levels in samples incubated in the presence of arachidonic acid and ethanol vehicle.

### Assay of the activity of purified human COX-2

The enzyme activity is measured using a chromogenic assay based on the oxidation of N,N,N',N'-tetramethyl-p-phenylenediamine (TMPD) during the reduction of PGG₂ to PGH₂ by COX-2 (Copeland et al. (1994) Proc. Natl. Acad. Sci. 91, 11202-11206).

Recombinant human COX-2 is purified from Sf9 cells as previously described (Percival et al (1994) Arch. Biochem. Biophys. 15, 111-118). The assay mixture (180 µL) contains 100 mM sodium phosphate, pH 6.5, 2 mM genapol X-100, 1 µM hematin, 1 mg/ml gelatin , 80-100 units of purified enzyme (One unit of enzyme is defined as the amount of enzyme required to produce an O.D. change of 0.001/min at 610 nm) and 4 µL of the test compound in DMSO. The mixture is pre-incubated at room temperature (22°C) for 15 minutes prior to initiation of the enzymatic reaction by the addition of 20 µL of a sonicated solution of 1 mM arachidonic acid (AA) and 1 mM TMPD in assay buffer (without enzyme or hematin). The enzymatic activity is measured by estimation of the initial velocity of TMPD oxidation over the first 36 sec of the reaction. A non-specific rate of oxidation is observed in the absence of enzyme (0.007 - 0.010 O.D. /min) and is subtracted before the calculation of the % inhibition. IC₅₀ values are derived from 4-parameter least squares non-linear regression analysis of the log-dose vs % inhibition plot.

### HUMAN WHOLE BLOOD ASSAY

### Rationale

Human whole blood provides a protein and cell-rich milieu appropriate for the study of biochemical efficacy of anti-inflammatory compounds such as selective COX-2 inhibitors. Studies have shown that normal human blood does not contain the COX-2 enzyme. This is consistent with the observation that COX-2 inhibitors have no effect on PGE₂ production in normal blood. These inhibitors are active only after incubation of human whole blood with LPS, which induces COX-2. This assay can be used to evaluate the inhibitory effect of selective COX-2 inhibitors on PGE₂ production. As well, platelets in whole blood contain a large amount of the COX-1 enzyme. Immediately following blood clotting, platelets are activated through a thrombin-mediated mechanism. This reaction results in the production of thromboxane B₂ (TxB₂) via activation of COX-1. Thus, the effect of test compounds on TxB₂ levels following blood clotting can be examined and used as an index for COX-1 activity. Therefore, the degree of selectivity by the test compound can be determined by measuring the levels of PGE₂ after LPS induction (COX-2) and TxB₂ following blood clotting (COX-1) in the same assay.

### Method

### A. COX-2 (LPS-induced PGE₂ production)

Fresh blood is collected in heparinized tubes by venipuncture from both male and female volunteers. The subjects have no apparent inflammatory conditions and have not taken any NSAIDs for at least 7 days prior to blood collection. Plasma is immediately obtained from a 2mL blood aliquot to use as blank (basal levels of PGE₂). The remaining blood is incubated with LPS (100 µg/ml final concentration, Sigma Chem, #L-2630 from E. coli; diluted in 0.1% BSA (Phosphate buffered saline) for 5 minutes at room temperature. Five hundred µL aliquots of blood are incubated with either 2µL of vehicle (DMSO) or 2µL of a test compound at final concentrations varying from 10nM to 30µM for 24 hours at 37°C. At the end of the incubation, the blood is centrifuged at 12,000 x g for 5 minutes to obtain plasma. A 100µL aliquot of plasma is mixed with 400µL of methanol for protein precipitation. The supernatant is obtained and is assayed for PGE₂ using a radioimmunoassay kit (Amersham, RPA#530) after conversion of PGE₂ to its methyl oximate derivative according to the manufacturer's procedure.

### B. COX-1 (Clotting-induced TxB₂ production)

Fresh blood is collected into vacutainers containing no anticoagulants. Aliquots of 500µL are immediately transferred to siliconized microcentrifuge tubes preloaded with 2µL of either DMSO or a test compound at final concentrations varying from 10nM to 30µM. The tubes are vortexed and incubated at 37°C for 1 hour to allow blood to clot. At the end of incubation, serum is obtained by centrifugation (12,000 x g for 5 min.). A 100µL aliquot of serum is mixed with 400µL of methanol for protein precipitation. The supernatant is obtained and is assayed for TxB₂ using a enzyme immunoassay kit (Cayman, #519031) according to the manufacturer's instruction.

### RAT PAW EDEMA ASSAY

### Protocol

Male Sprague-Dawley rats (150-200 g) are fasted overnight and are given po, either vehicle (1% methocel or 5% Tween 80) or a test compound. One hr later, a line is drawn using a permanent marker at the level above the ankle in one hind paw to define the area of the paw to be monitored. The paw volume (V₀) is measured using a plethysmometer (Ugo-Basile, Italy) based on the principle of water displacement. The animals are then injected subplantarly with 50 µl of 1% carrageenan solution in saline (FMC Corp, Maine) into the paw using an insulin syringe with a 25-gauge needle (i.e. 500 µg carrageenan per paw). Three hr later, the paw volume (V₃) is measured and the increases in paw volume (V₃ - V_{O}) are calculated. The animals are sacrificed by CO₂ asphyxiation and the absence or presence of stomach lesions scored. Data is compared with the vehicle-control values and percent inhibition calculated. All treatment groups are coded to eliminate observer bias.

### NSAID-INDUCED GASTROPATHY IN RATS

### Rationale

The major side effect of conventional NSAIDs is their ability to produce gastric lesions in man. This action is believed to be caused by inhibition of Cox-1 in the gastrointestinal tract. Rats are particularly sensitive to the actions of NSAIDs. In fact, rat models have been used commonly in the past to evaluate the gastrointestinal side effects of current conventional NSAIDs. In the present assay, NSAID-induced gastrointestinal damage is observed by measuring fecal ⁵¹Cr excretion after systemic injection of ⁵¹Cr-labeled red blood cells. Fecal ⁵¹Cr excretion is a well-established and sensitive technique to detect gastrointestinal integrity in animals and man.

### Methods

Male Sprague Dawley rats (150 - 200 g) are administered orally a test compound either once (acute dosing) or b.i.d. for 5 days (chronic dosing). Immediately after the administration of the last dose, the rats are injected via a tail vein with 0.5 mL of ⁵¹Cr-labeled red blood cells from a donor rat. The animals are placed individually in metabolism cages with food and water ad *lib*. Feces are collected for a 48 h period and ⁵¹Cr fecal excretion is calculated as a percent of total injected dose. ⁵¹Cr-labeled red blood cells are prepared using the following procedures. Ten mL of blood is collected in heparinized tubes via the vena cava from a donor rat. Plasma is removed by centrifugation and replenished with equal volume of HBSS. The red blood cells are incubated with 400 Ci of sodium ⁵¹chromate for 30 min. at 37° C. At the end of the incubation, the red blood cells are washed twice with 20 mL HBSS to remove free sodium ⁵¹chromate. The red blood cells are finally reconstituted in 10 mL HBSS and 0.5 mL of the solution (about 20 Ci) is injected per rat.

### PROTEIN-LOSING GASTROPATHY IN SQUIRREL MONKEYS

### Rationale

Protein-losing gastropathy (manifested as appearance of circulating cells and plasma proteins in the GI tract) is a significant and dose-limiting adverse response to standard non-steroidal anti-inflammatory drugs (NSAIDs). This can be quantitatively assessed by intravenous administration of ⁵¹CrCl₃ solution. This isotopic ion can avidly bind to cell and serum globins and cell endoplasmic reticulum. Measurement of radioactivity appearing in feces collected for 24 h after administration of the isotope thus provides a sensitive and quantitative index of protein-losing gastropathy.

### Methods

Groups of male squirrel monkeys (0.8 to 1.4 kg) are treated by gavage with either 1% methocell or 5% Tween 80 in H₂0 vehicles, (3mL/kg b.i.d.) or test compounds at doses from 1 - 100 mg/kg b.i.d. for 5 days. Intravenous ⁵¹Cr (5Ci/kg in 1 ml/kg phosphate buffer saline (PBS)) is administered 1 h after the last drug/vehicle dose, and feces collected for 24 h in a metabolism cage and assessed for excreted ⁵¹Cr by gamma-counting. Venous blood is sampled 1 h and 8 h after the last drug dose, and plasma concentrations of drug measured by RP-HPLC.

### LPS-Induced Pyrexia in Conscious Rats

Male Sprague-Dawley rats (150 - 200 g) were fasted for 16 - 18 h before use. At approximately 9:30 a.m., the animals were placed temporarily in plexiglass restrainers and their baseline rectal temperature was recorded using a flexible temperature probe (YSI series 400) connected to a digital thermometer (Model 08502, Cole Parmer). The same probe and thermometer were used for all animals to reduce experimental error. The animals were returned to their cages after the temperature measurements. At time zero, the rats were injected intraperitoneally with either saline or LPS (2 mg/kg, Sigma Chem) and the rectal temperature was remeasured at 5, 6 and 7 h following LPS injection. After the measurement at 5 h, when the increase in temperature had reached a plateau, the LPS-injected rats were given either the vehicle (1% methocel) or a test compound orally to determine whether the compound could reverse the pyrexia. Percent reversal of the pyrexia was calculated using the rectal temperature obtained at 7 h in the control (vehicle-treated) group as the reference (zero reversal) point. Complete reversal of pyrexia to the pre-LPS baseline value is taken as 100%.

### LPS-Induced Pyrexia in Conscious Squirrel Monkeys

Temperature probes were surgically implanted under the abdominal skin in a group of squirrel monkeys (*Saimiri sciureus*) (1.0 - 1.7 kg). This allows for the monitoring of body temperature in conscious, unrestrained monkeys by a telemetric sensing system (Data Sciences International, Minnesota). The animals were fasted and were placed in individual cages for acclimatization 13 - 14 h before use. Electronic receivers were installed on the side of the cages which pick up signals from the implanted temperature probes. At approximately 9:00 a.m. on the day of the experiment, the monkeys were restrained temporarily in training chairs and were given a bolus I.V. injection of LPS, (6 mg/kg, dissolved in sterile saline). The animals were returned to their cages and body temperature was recorded continuously every 5 min. Two h after injection of LPS, when the body temperature had increased by 1.5 - 2° C, the monkeys were dosed orally with either vehicle (1% methocel) or a test compound (3 mg/kg). One hundred minutes later, the difference between the body temperature and the baseline value was determined. Percent inhibition was calculated taking the value in the control group as 0% inhibition.

### Acute Inflammatory Hyperalgesia Induced by Carrageenan in Rats

Experiments were performed using male Sprague Dawley rats (90-110g). Hyperalgesia to mechanical compression of the hind paw was induced by intraplantar injection of carrageenan (4.5 mg into one hind paw) 3 h previously. Control animals received an equivalent volume of saline (0.15 ml intraplantar). A test compound (0.3-30 mg/kg, suspended in 0.5% methocel in distilled water) or vehicle (0.5% methocel) was administered orally (2ml/kg) 2 h after carrageenan. The vocalisation response to compression of the hind paw was measured 1 h later using a Ugo Basile algesiometer.

Statistical analysis for carrageenan-induced hyperalgesia was performed using one-way ANOVA (BMDP Statistical Software Inc.). Hyperalgesia was determined by subtracting the vocalisation threshold in saline injected rats from that obtained in animals injected with carrageenan. Hyperalgesia scores for drug-treated rats were expressed as a percentage of this response. ID₅₀ values (the dose producing 50% of the maximum observed response) were then calculated by nonlinear least squares regression analysis of mean data using GraFit (Erithacus Software).

### Adjuvant-Induced Arthritis in Rats

Seventy, 6.5-7.5 week old, female Lewis rats (body weight ∼146-170 g) were weighed, ear marked, and assigned to groups (a negative control group in which arthritis was not induced, a vehicle control group, a positive control group administered indomethacin at a total daily dose of 1 mg/kg and four groups administered with a test compound at total daily doses of 0.10-3.0 mg/kg) such that the body weights were equivalent within each group. Six groups of 10 rats each were injected into a hind paw with 0.5 mg of *Mycobacterium butyricum* in 0.1 ml of light mineral oil (adjuvant), and a negative control group of 10 rats was not injected with adjuvant. Body weights, contralateral paw volumes (determined by mercury displacement plethysmography) and lateral radiographs (obtained under Ketamine and Xylazine anesthesia) were determined before (day -1) and 21 days following adjuvant injection, and primary paw volumes were determined before (day -1) and on days 4 and 21 following adjuvant injection. The rats were anesthetized with an intramuscular injection of 0.03 - 0.1 ml of a combination of Ketamine (87 mg/kg) and Xylazine (13 mg/kg) for radiographs and injection of adjuvant. The radiographs were made of both hind paws on day 0 and day 21 using the Faxitron (45 kVp, 30 seconds) and Kodak X-OMAT TL film, and were developed in an automatic processor. Radiographs were evaluated for changes in the soft and hard tissues by an investigator who was blinded to experimental treatment. The following radiographic changes were graded numerically according to severity: increased soft issue volume (0-4), narrowing or widening of joint spaces (0-5) subchondral erosion (0-3), periosteal reaction (0-4), osteolysis (0-4) subluxation (0-3), and degenerative joint changes (0-3). Specific criteria were used to establish the numerical grade of severity for each radiographic change. The maximum possible score per foot was 26. A test compound at total daily doses of 0.1, 0.3, 1, and 3 mg/kg/day, Indomethacin at a total daily dose of 1 mg/kg/day, or vehicle (0.5% methocel in sterile water) were administered per os b.i.d. beginning post injection of adjuvaut and continuing for 21 days. The compounds were prepared weekly, refrigerated in the dark until used, and vortex mixed immediately prior to administration.

Two-factor ('treatment' and 'time') analysis of variance with repeated measures on 'time' were applied to the % changes for body weight and foot volumes and to the rank-transformed radiographic total scores. A *post hoc* Dunnett's test was conducted to compare the effect of treatments to vehicle. A one-way analysis of variance was applied to the thymic and spleen weights followed by the Dunnett's test to compare the effect of treatments to vehicle. Dose-response curves for % inhibition in foot volumes on days 4, 14 and 21 were fitted by a 4-parameter logistic function using a nonlinear least squares' regression. ID₅₀ was defined as the dose corresponding to a 50% reduction from the vehicle and was derived by interpolation from the fitted 4-parameter equation.

### Representative Biological Data

Compounds of the present invention are inhibitors of COX-2 and are thereby useful in the treatment of COX-2 mediated diseases as enumerated above. The activities of compound A against cyclooxygenase may be seen in the representative results shown below in the Table. In the assays, inhibition is determined by measuring the amount of a prostaglandin synthesized in the presence and absence of a putative inhibitor. The IC₅₀ values represent the concentration of putative inhibitor required to lower prostaglandin synthesis to 50% of that obtained as compared to the uninhibited control. The ED₅₀ values in the rat paw edema assay represent the dose of compound required to reduce edema formation by 50% as compared to the vehicle control. Shown also are the corresponding biological activities of Compound B, which compound is disclosed in WO 95/00501, Example 7 and US 5,536,752.

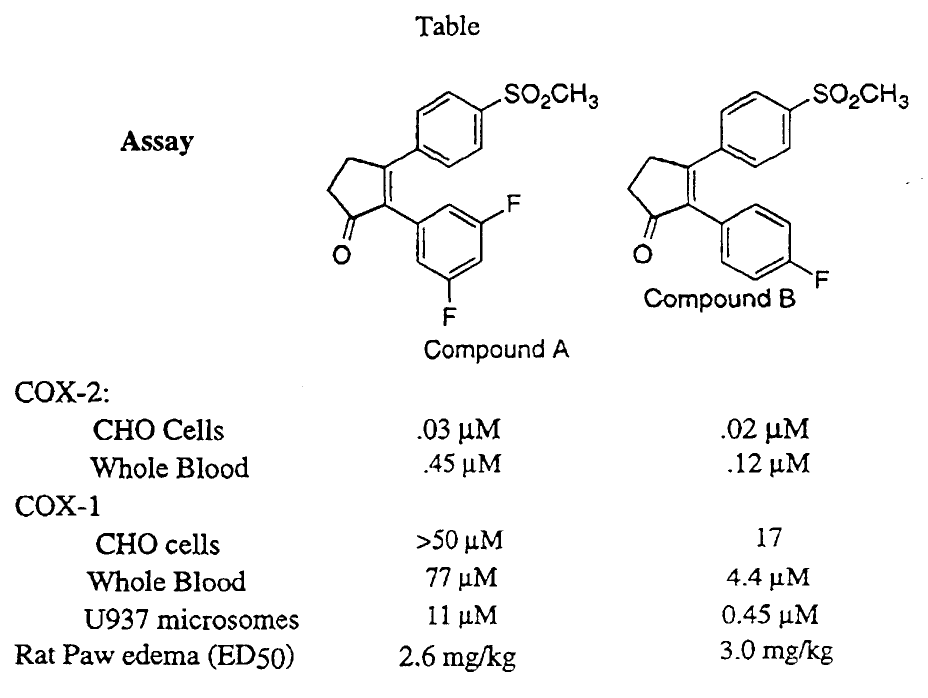

The invention will now be illustrated by the following non-limiting examples in which, unless stated otherwise:
(i) all operations were carried out at room or ambient temperature, that is, at a temperature in the range 18-25°C;
(ii) evaporation of solvent was carried out using a rotary evaporator under reduced pressure (600-4000 pascals: 4.5-30 mm Hg) with a bath temperature of up to 60°C;
(iii) the course of reactions was followed by thin layer chromatography (TLC) and reaction times are given for illustration only;
(iv) melting points are uncorrected and 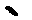 d' indicates decomposition; the melting points given are those obtained for the materials prepared as described; polymorphism may result in isolation of materials with different melting points in some preparations;
(v) the structure and purity of all final products were assured by at least one of the following techniques: TLC, mass spectrometry, nuclear magnetic resonance (NMR) spectrometry or microanalytical data;
(vi) yields are given for illustration only;
(vii) when given, NMR data is in the form of delta (δ) values for major diagnostic protons, given in parts per million (ppm) relative to tetramethylsilane (TMS) as internal standard, determined at 300 MHz or 400 MHz using the indicated solvent; conventional abbreviations used for signal shape are: s. singlet; d. doublet; t. triplet; m. multiplet; br. broad; etc.: in addition "Ar" signifies an aromatic signal;
(viii) chemical symbols have their usual meanings; the following abbreviations have also been used v (volume), w (weight), b.p. (boiling point), M.P. (melting point), L (liter(s)), mL (milliliters), g (gram(s)), mg (milligrams(s)), mol (moles), mmol (millimoles), eq (equivalent(s)).

Compound A can be prepared according to the following methods Methods of Synthesis

### Method A

Cyclopentenone (**II**) may be halogenated with bromine or iodine followed by treatment with a base to give **III** (see Organic Syntheses **61** 65). 3,5-Difluorophenylboronic acid may then be added via a palladium-catalyzed coupling reaction to form **IV**. 4-Bromothioanisole may be metalated with nBuLi or magnesium, and then treated with **IV** to give the alcohol **V**. Oxidation with allylic transposition may then be accomplished using an oxidant such as PDC to give cyclopentenone **VI**. Sulfide oxidation using an oxidant such as H₂O₂ in the presence of a tungstate catalyst, MMPP, or mCPBA then provides sulfone **Ia**. Alternatively, **VI** may be converted to sulfonamide **Ib** as described in U.S. Patent 5,474,995, Method A.

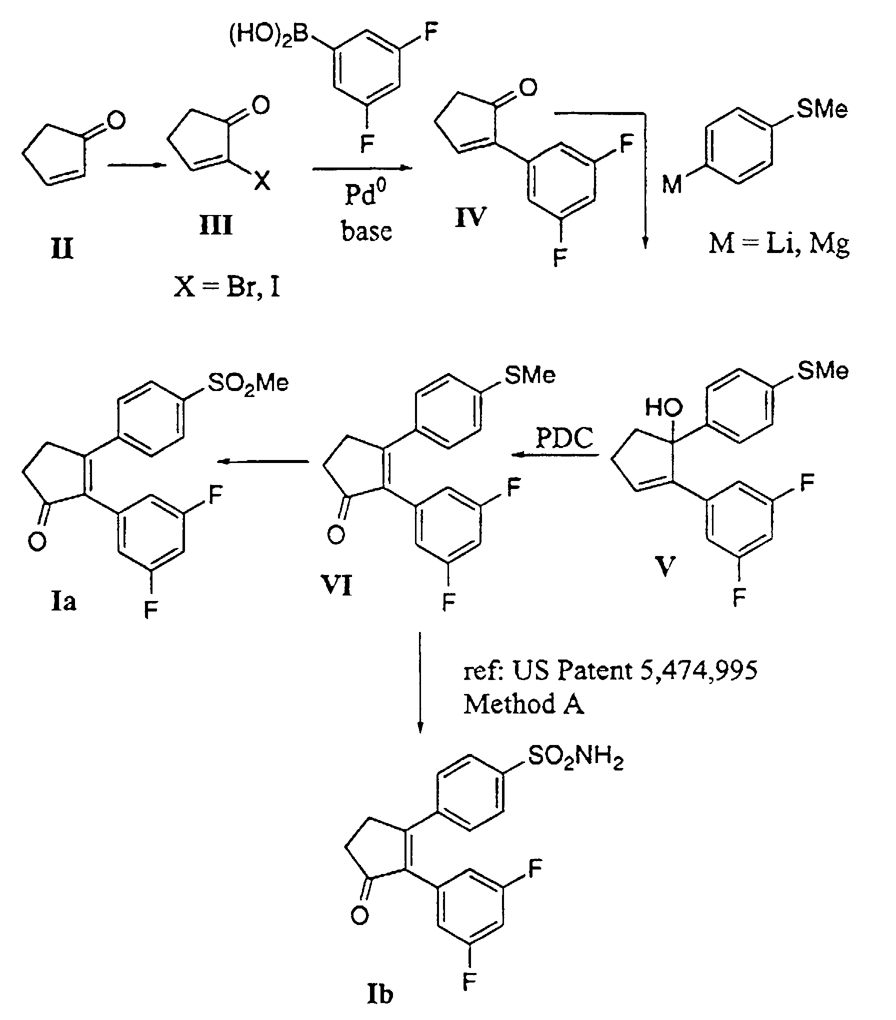

### Method B

To bromocyclopentenone **IIIa** is added lithio or magnesium thioanisole to give tertiary alcohol **VII**. Oxidation with a reagent such as PDC then provides the ketone **VIII**. The sulfide may be oxidized at this point using an oxidant such as H₂O₂ in the presence of a tungstate catalyst, MMPP, or mCPBA to give the sulfone **IX**. Palladium-catalyzed coupling of 3,5-difluorophenylboronic acid then provides **Ia**.

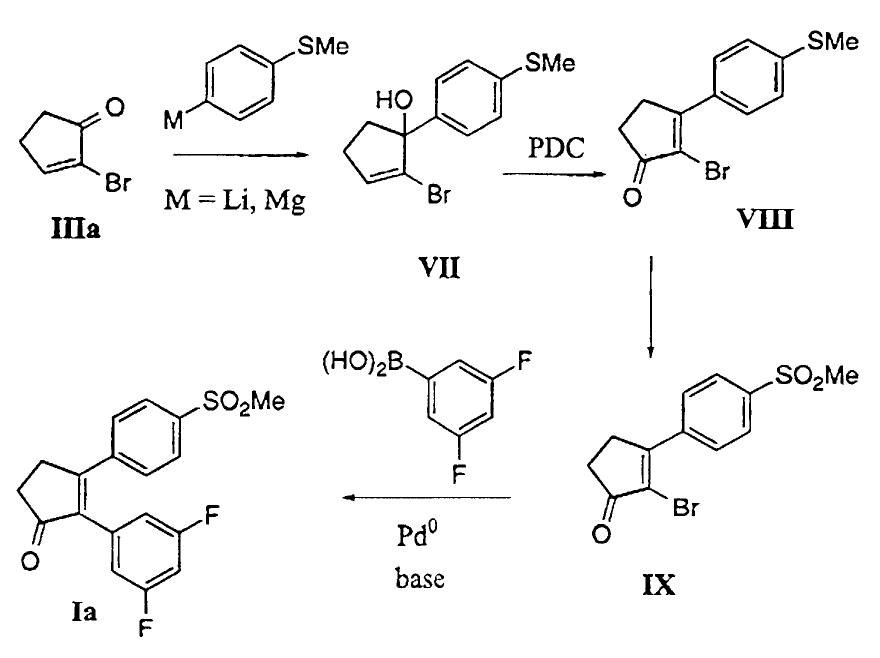

### Method C

3-Ethoxycyclopentenone (**X**) may be brominated followed by treatment with a base to give **XI** (see Organic Syntheses **61** 65). To bromocyclopentenone **XI** is added lithio or magnesium thioanisole followed by acid rearrangement of the intermediate addition product to give **VIII**. The sulfide may be oxidized at this point using a oxidant such as H₂O₂ in the presence of a tungstate catalyst, MMPP, or mCPBA to give the sulfone **IX**. Palladium-catalyzed coupling of 3,5-difluorophenylboronic acid then provides **Ia**.

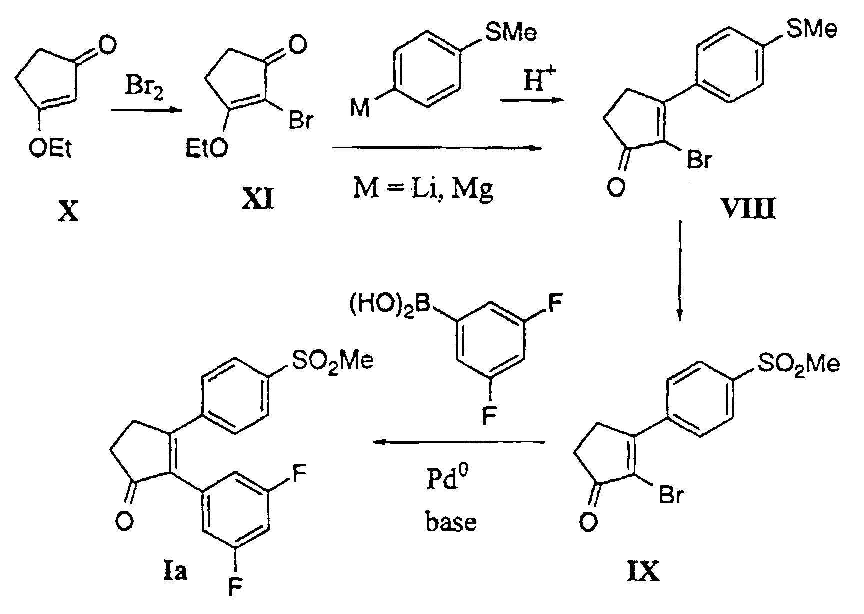

### EXAMPLE 1

### 2-(3,5-DIFLUOROPHENYL)-3-(4-(METHYLSULFONYL)PHENYL)-2-CYCLOPENTEN-1-ONE (COMPOUND A)

### Step 1 3-(4-(Methylthio)phenyl)-2-cyclopenten-1-one

To a -65°C solution of 4-bromothioanisole (61 g, 0.3 mol) in THF (800 mL) was added a solution of n-BuLi (2.3 M in hexanes, 120 mL) at a rate so as to maintain an internal temperature below -55°C. The resulting slush was stirred at -65°C for 2 h, then treated with a solution of 3-ethoxy-2-cyclopenten-1-one (35 g, 0.28 mmol) in THF (50 mL). After 30 min at -65°C, the solution was warmed to 0°C and quenched with sat. aq. NH₄Cl (400 mL). The product was extracted with 3 x 1L of EtOAc and the combined extracts were dried over MgSO₄ and concentrated. The resulting material was swished in 200 mL of 1:1 EtOAc/hexanes to provide 45 g of the title compound as a white solid.

### Step 2 2-Bromo-3-(4-(methylthio)phenyl)-2-cyclopenten-1-one

To a suspension of 3-(4-(methylthiol)phenyl)-2-cyclopenten-1-one (9.64 g, 47.2 mmol) in CCl₄ (200 mL) was added a solution of bromine (5 mL) in CCl₄ (30 mL) over 20 min. The resulting orange suspension was stirred for 1.5 h, then cooled in an ice-bath, and triethylamine (14 mL, 100 mmol) was added. After 30 min, the mixture was quenched with 1M HCl (300 mL) and extracted with CH₂Cl₂. The organic phase was washed with brine, filtered through cotton and evaporated. Purification by silica gel chromatography, eluting with 90% CH₂Cl₂/hexanes gave the title compound (7.13 g).

### Step 3 2-Bromo-3-(4-(methylsulfonyl)phenyl)-2-cyclopenten-1-one

To a 0 °C solution of 2-bromo-3-(4-(methylthio)phenyl)-2-cyclopenten-1-one (5.73 g, 20.2 mmol) in CH₂Cl₂ (100 mL) and MeOH (50 mL) was added MMPP (19 g, 30.7 mmol). The mixture was stirred 5 h at r.t., then concentrated. The residue was partitioned between sat. aq. NaHCO₃ and CH₂Cl₂. The organic phase was washed with brine, filtered through cotton and evaporated. The resulting solid was swished in CH₂Cl₂/hexanes to provide the title compound (5.57 g).

### Step 4 2-(3,5-Difluorophenyl)-3-(4-(methylsulfonyl)phenyl)-2-cyclopenten-1-one

To a mixture of 2-bromo-3-(4-(methylsulfonyl)phenyl)-2-cyclopenten-1-one (664 mg, 2.11 mmol), 3,5-difluorophenylboronic acid (832 mg, 5.27 mmol), tris(dibenzylideneacetone)dipalladium(0) (83 mg, 0.09 mmol), and triphenylphosphine (96 mg, 0.36 mmol) was added 40 mL of 3:1:1 toluene:*n*-propanol:water and the mixture was purged with nitrogen. After stirring 10 min, diethylamine (1.1 mL, 10.6 mmol) was added and the solution was heated to reflux for 4 h. The mixture was cooled and partitioned between 1M NaOH and EtOAc. The organic phase was washed with water, 1M HCl and brine, and dried over MgSO₄. Purification by silica gel chromatography, eluting with 50% CH₂Cl₂/hexanes, followed by crystallization from CH₂Cl₂/ether/hexanes gave the title compound (223 mg).
¹H NMR (CD₃COCD₃) δ 7.95 (2H, d), 7.65 (2H, d), 6.98 (1H, m), 6.82 (2H, m), 3.19 (2H, m), 3.15 (3H, s), 2.68 (2H, m).

### EXAMPLE 1A

### Step 1 2-Bromo-2-cyclopenten-1-one

To a 0 °C solution of 2-cyclopenten-1-one (125 g, 1.52 mol) in CCl₄ (1.2 L) in a three-neck flask equipped with an overhead stirrer was added a solution of bromine (269 g, 1.68 mol) in CCl₄ (400 mL) dropwise over 4 h, maintaining an internal temperature <2 °C. A solution of Et₃N (310 mL, 2.22 mol) in CCl₄ (200 mL) was then added dropwise over 1.5 h, maintaining an internal temperature <10 °C. The resulting suspension was warmed to r.t. for 1 h, then cooled to 0 °C and filtered. The filtrate was washed with two 700 mL portions of 3M HCl and 500 mL of brine, then filtered through cotton. Concentration provided 228 g of an orange oil which was crystalized from 150 mL of 2:1 hexane: ether to provide 191 g of the title compound.
¹H NMR (CD₃COCD₃) δ 7.94 (1H, t), 2.72 (2H, m), 2.46 (2H, m).

### Step 2 2-Bromo-3-(4-(methylthio)phenyl)-2-cyclopenten-1-one

To a -78 °C solution of 4-bromothioanisole (35.1 g, 173 mmol) in THF (500 mL) was added nBuLi (1.6M in hexanes, 107.5 mL, 172 mmol). The solution was stirred for 45 min, then a solution of 2-bromo-2-cyclopenten-1-one (25.4 g, 158 mmol) in THF (150 mL) was added and the mixture was allowed to warm to 0 °C and was quenched with saturated aqueous NH₄Cl. The majority of the solvent was removed *in vacuo* and the residue was suspended in water and extracted with two portions of EtOAc. The organic layers were washed with brine, dried over MgSO₄, filtered and concentrated. This material was dissolved in DMF (300 mL), cooled to 0 °C and treated with PDC (72.4 g, 192 mmol). The resulting mixture was warmed to r.t. and stirred for 2 h, then poured into water (1.2 L) and extracted with two 500 mL portions of EtOAc. The organic layers were washed with brine, dried over MgSO₄, filtered and concentrated to give the title compound as a light brown solid which was used directly in the next step.

### Step 3 2-Bromo-3-(4-(methylsulfonyl)phenyl)-2-cyclopenten-1-one

To a 0 °C solution of 2-bromo-3-(4-(thiomethyl)phenyl)-2-cyclopenten-1-one in 2:1 CH₂Cl₂/MeOH (500 mL) was added MMPP (100 g). The mixture was stirred at r.t. overnight, then concentrated and partitioned between saturated NaHCO₃, 1M Na₂S₂O₃ and CH₂Cl₂. The aqueous layer was extracted with CH₂Cl₂, and the combined organics were washed with brine, filtered through cotton and evaporated. The resulting solid was swished in CH₂Cl₂/ether to provide 23 g of the title compound.
¹H NMR (CD₃COCD₃) δ 8.12 (4H, m), 3.22 (2H, m), 3.20 (3H, s), 2.69 (2H, m).

### Step 4 3,5-Difluorophenylboronic acid

To a -78 °C solution of 1-bromo-3,5-difluorobenzene (50 g, 0.26 mol) in ether (860 mL) was added nBULi (2.4 M in hexanes, 108 mL, 0.26 mol) dropwise over 20 min. The resulting solution was stirred for 10 min, then treated with triisopropylborate (61 mL, 0.27 mol). The reaction mixture was warmed to 0 °C for 30 min, then quenched with 1 M HCl. After stirring 30 min, the mixture was partitioned between ethyl acetate and water. The organic layers were washed with brine, dried over MgSO₄, filtered, and evaporated. The resulting product was dried under high vacuum overnight to give 38 g of the title compound.
¹H NMR (CD₃COCD₃) δ 7.50 (2H, br s), 7.40 (2H, m), 7.04 (1H, m),

### Step 5 2-(3,5-Difluorophenyl)-3-(4-(methylsulfonyl)phenyl)-2-cyclopenten-1-one

A mixture of 2-bromo-3-(4-(methylsulfonyl)phenyl)-2-cyclopenten-1-one (52.2 g, 166 mmol), 3,5-difluorophenylboronic acid (31.9 g, 202 mmol), tris(dibenzylideneacetone)dipalladium(0) (3.3 g, 3.6 mmol), and triphenylphosphine (1.89 g, 7.2 mmol) were dissolved in toluene (800 mL) and nPrOH (250 mL) and degassed. After stirring for 10 min, diethylamine (21 mL, 203 mmol) and water (250 mL) was added. The mixture degassed again, heated to reflux for 1h, then cooled and poured into EtOAc (2L). The aqueous layer was separated, and the organic layer was washed with 0.2N NaOH (500 mL), 0.5N HCl (500 mL) and brine. The organic phase was then dried over MgSO₄, filtered through celite and evaporated. The resulting light brown solid was swished in hot EtOAc (250 mL), cooled and filtered to give 45.8 g of the title compound, m.p. 174-175 °C.
H NMR (CD₃COCD₃) δ 7.95 (2H, m), 7.66 (2H, m), 6.98 (1H, m), 6.80 (2H, m), 3.17 (2H, m), 3.12 (3H, s), 2.68 (2H, m).

### B. An alternative method of preparing 2-bromo-3-(4-(methylsulfonyl)phenyl)-2-cyclopenten-1-one (Example 1, Step 3 has been developed as follows:

### Step 1 2-Bromo-3-ethoxy-2-cyclopenten-1-one

To a -15 °C solution of 3-ethoxy-2-cyclopenten-1-one (165g, 1.30 mol) in 1.8L of CHCl₃ was added a solution of bromine (219 g, 1.37 mol) in CHCl₃ (200 mL) via dropping funnel over 1.5h to give a thick yellow slurry. After 30 min additional stirring, a solution of Et₃N (210 mL, 1.5 mol) in CHCl₃ (300 mL) was added via dropping funnel over 30 min. The resulting mixture was concentrated, suspended in EtOAc and filtered through a pad of 2 cm celite over 1 cm silica gel. The filtrate was concentrated, and the resulting material recrystalized from 4:1 ether/hexanes to provide 245 g of the title compound.
H NMR (CD₃COCD₃) δ 4.43 (2H, q), 2.95 (2H, m), 2.47 (2H, m), 1.39 (3H, t).

### Step 2 2-Bromo-3-(4-methylthio)phenyl)-2-cyclopenten-1-one

To a -78 °C solution of 4-bromothioanisole (253 g, 1.25 mol) in THF (5 L) was added nBuLi (2.5 M in hexanes, 500 mL, 1.25 mol) over 20 min to give a thick slurry which was stirred 2h at -78 °C. A solution of 2-bromo-2-cyclopenten-1-one (242 g, 1.18 mol) in THF (1 L) was then added via cannula and the mixture was allowed to warm to -10 °C. 6N HCl (430 mL) was then added and the resulting mixture stirred 20 min. EtOAc (3L) was added and the aqueous phase was separated. The organic layer was concentrated. The residue was suspended in ether and filtered to give 287 g of the title compound.
¹H NMR (CD₃COCD₃) δ 8.12 (4H, m), 3.22 (2H, m), 3.20 (3H, s), 2.69 (2H, m).

The following example pharmaceutical formulation are illustrative of the invention:

### Step 3 2-Bromo-3-(4-(methylsulfonyl)phenyl)-2-cyclopenten-1-one

To a room temperature suspension of 2-bromo-3-(4-(methylthio)phenyl)-2-cyclopenten-1-one (67.4 g, 238 mmol) in EtOAc (500 mL) and CH2Cl2 (100 mL) was added NaWO4 2H2O (3.1 g, 9 mmol) and Aliquat 336 (12 g, 30 mmol). 10 mL of 30% H2O2 was added and the mixture warmed to 40°C to initiate the oxidation. 85 mL of H2O2 was then added dropwise over 20 min with external cooling to maintain an internal temperature of approximately 50°C. The dark reaction mixture was then maintained at 40°C for 1.5 h. The aqueous layer was separated and the organic phase was washed twice with warm water. The organic phase was then concentrated to 500 mL and the precipitate was filtered and washed with ether to give 52.2 g of the title compound as a white solid.

### EXAMPLE 2

| Wet granulated tablet composition | |
|---|---|
| **Amount per tablet** | **Ingredient** |
| 25 mg | COX-2 Inhibitor |
| 79.7 mg | Microcrystalline cellulose |
| 79.7 mg | Lactose monohydrate |
| 6 mg | Hydroxypropyl cellulose |
| 8 mg | Croscarmellose sodium |
| 1 mg | Magnesium stearate |

Tablet dose strengths of between 5 and 125 mg can be accomodated by varying total tablet weight, and the ratio of the first three ingredients. Generally it is preferable to maintain a 1:1 ratio for microcrystalline cellulose : lactose monohydrate. Varying amounts of up to 5 mg. of iron oxide may be added to this or the other compositions if a yellow or orange color is desired.

### EXAMPLE 2a

| Wet granulated tablet composition | |
|---|---|
| **Amount per tablet** | **Ingredient** |
| 125 mg | COX-2 Inhibitor |
| 425 mg | Microcrystalline cellulose |
| 425 mg | Lactose monohydrate |
| 10 mg | Hydroxypropyl cellulose |
| 14 mg | Croscarmellose sodium |
| 1 mg | Magnesium stearate |

### EXAMPLE 2b

| Wet granulated tablet composition | |
|---|---|
| **Amount per tablet** | **Ingredient** |
| 10 mg | COX-2 Inhibitor |
| 87.2 mg | Microcrystalline cellulose |
| 87.2 mg | Lactose monohydrate |
| 6 mg | Hydroxypropyl cellulose |
| 8 mg | Croscarmellose sodium |
| 1 mg | Magnesium stearate |

### EXAMPLE 2c

| Wet granulated tablet composition | |
|---|---|
| **Amount per tablet** | **Ingredient** |
| 5 mg | COX-2 Inhibitor |
| 89.7 mg | Microcrystalline cellulose |
| 89.7 mg | Lactose monohydrate |
| 6 mg | Hydroxypropyl cellulose |
| 8 mg | Croscarmellose sodium |
| 1 mg | Magnesium stearate |

### EXAMPLE 3

| Directly compressed tablet composition | |
|---|---|
| **Amount per tablet** | **Ingredient** |
| 25 mg | COX-2 Inhibitor |
| 106.9 mg | Microcrystalline cellulose |
| 106.9 mg | Lactose anhydrate |
| 7.5 mg | Croscarmellose sodium |
| 3.7 mg | Magnesium stearate |

Tablet dose strengths of between 5 and 125 mg can be accomodated by varying total tablet weight, and the ratio of the first three ingredients. Generally it is preferable to maintain a 1:1 ratio for microcrystalline cellulose : lactose monohydrate.

### EXAMPLE 3a

| Directly compressed tablet composition | |
|---|---|
| **Amount per tablet** | **Ingredient** |
| 125 mg | COX-2 Inhibitor |
| 425 mg | Microcrystalline cellulose |
| 425 mg | Lactose anhydrate |
| 15 mg | Croscarmellose sodium |
| 10 mg | Magnesium stearate |

### EXAMPLE 3b

| Directly compressed tablet composition | |
|---|---|
| **Amount per tablet** | **Ingredient** |
| 10 mg | COX-2 Inhibitor |
| 42.5 mg | Microcrystalline cellulose |
| 42.5 mg | Lactose anhydrate |
| 4 mg | Croscarmellose sodium |
| 1 mg | Magnesium stearate |

### EXAMPLE 3c

| Directly compressed tablet composition | |
|---|---|
| **Amount per tablet** | **Ingredient** |
| 5 mg | COX-2 Inhibitor |
| 45 mg | Microcrystalline cellulose |
| 45 mg | Lactose anhydrate |
| 4 mg | Croscarmellose sodium |
| 1 mg | Magnesium stearate |

### EXAMPLE 4

| Hard gelatin capsule composition | |
|---|---|
| **Amount per capsule** | **Ingredient** |
| 25 mg | COX-2 Inhibitor |
| 37 mg | Microcrystalline cellulose |
| 37 mg | Lactose anhydrate |
| 1 mg | Magnesium stearate |
| 1 capsule | Hard gelatin capsule |

Capsule dose strengths of between 1 and 50 mg can be accomodated by varying total fill weight, and the ratio of the first three ingredients. Generally it is preferable to maintain a 1:1 ratio for microcrystalline cellulose : lactose monohydrate.

### EXAMPLE 5

| Oral solution | |
|---|---|
| **Amount per 5 mL dose** | **Ingredient** |
| 50 mg | COX-2 Inhibitor |
| to 5 mL with Polyethylene oxide 400 | |

Solution dose strengths of between 1 and 50 mg/5mL can be accommodated by varying the ratio of the two ingredients.

### EXAMPLE 6

| Oral suspension | |
|---|---|
| **Amount per 5 mL dose** | **Ingredient** |
| 100 mg | COX-2 Inhibitor |
| 150 mg | Polyvinylpyrrolidone |
| 2.5 mg | Poly oxyethylene sorbitan monolaurate |
| 10 mg | Benzoic acid |
| to 5 mL with sorbitol solution (70%) | |

Suspension dose strengths of between 1 and 50 mg/5ml can be accomodated by varying the ratio of the first two ingredients.

### EXAMPLE 7

| Intravenous infusion | |
|---|---|
| **Amount per 200mL dose** | **Ingredient** |
| 1 mg | COX-2 inhibitor |
| 0.2 mg | Polyethylene oxide 400 |
| 1.8 mg | Sodium chloride |
| to 200mL | Purified water |

## Claims

1. A pharmaceutical composition for the treatment of cyclooxygenase-2 mediated diseases, said composition comprising a therapeutically effective amount of a compound which is 2-(3,5-difluorophenyl)-3-(4-(methylsulfonyl)phenyl)-2-cyclopenten-1-one and a pharmaceutically acceptable carrier.

2. A pharmaceutical composition comprising 2-(3,5-difluorophenyl)-3-(4-(methylsulfonyl)phenyl)-2-cyclopenten-1-one and a pharmaceutically acceptable carrier.

3. A pharmaceutical composition according to Claim 1 or 2 comprising 10 to 250 mg 2-(3,5-difluorophenyl)-3-(4-(methylsulfonyl)phenyl)-2-cyclopenten-1-one and a pharmaceutically acceptable carrier.

4. A composition according to Claim 1 or 2 comprising 10, 20, 30, 40 or 50 mg of 2-(3,5-difluorophenyl)-3-(4-methylsulfonyl)phenyl)-2-cyclopenten-1-one.

5. A composition according to Claim 1 or 2 comprising 60, 70, 80, 90, 100, 110, or 120 mg of 2-(3,5-difluorophenyl)-3-(4-methylsulfonyl)phenyl)-2-cyclopenten-1-one.

6. A composition according to Claim 1 or 2 comprising 60 to 120 mg of 2-(3,5-difluorophenyl)-3-(4- methylsulfonyl)phenyl)-2-cyclopenten-1-one.

7. A composition according to Claim 1 or 2 comprising 10 to 50 mg of 2-(3,5-difluorophenyl)-3-(4- methylsulfonyl)phenyl)-2-cyclopenten-1-one.

8. A method of treating an inflammatory disease susceptible to treatment with an non-steroidal anti-inflammatory agent comprising administration to a patient in need of such treatment 10 to 250 mg 2-(3,5-difluorophenyl)-3-(4-(methylsulfonyl)phenyl)-2-cyclopenten-1-one.

9. A method according to Claim 8 comprising administration to a patient in need of such treatment 10, 20, 30, 40 or 50 mg of 2-(3,5-difluorophenyl)-3-(4- methylsulfonyl)phenyl)-2-cyclopenten-1-one.

10. A method according to Claim 8 comprising administration to a patient in need of such treatment 60, 70, 80, 90, 100, 110, or 120 mg of 2-(3,5-difluorophenyl)-3-(4- methylsulfonyl)phenyl)-2-cyclopenten-1-one.

11. A method according to Claim 8 comprising administration to a patient in need of such treatment 60 to 120 mg of 2-(3,5-difluorophenyl)-3-(4- methylsulfonyl)phenyl)-2-cyclopenten-1-one.

12. A method according to Claim 8 comprising administration to a patient in need of such treatment 10 to 50 mg of 2-(3,5-difluorophenyl)-3(4- methylsulfonyl)phenyl)-2-cyclopenten-1-one.

13. A method according to Claim 8 for the treatment of non-chronic headache, pain or swelling.

14. A method according to Claim 8 for the treatment of osteoarthritis.

15. A method according to Claim 8 for the treatment of rheumatoid arthritis.

16. Use of 2-(3,5-difluorophenyl)-3-(4-(methylsulfonyl)phenyl)-2-cyclopenten-1-one in the manufacture of a medicament for the treatment of an inflammatory disease susceptible to treatment with a non-steroidal anti-inflammatory agent.

17. Use according to claim 16 of 10 to 250 mg 2-(3,5-difluorophenyl)-3-(4-(methylsulfonyl)phenyl)-2-cyclopenten-1-one in the manufacture of a dosage form of a medicament for the treatment of an inflammatory disease susceptible to treatment with a non-steroidal anti-inflammatory agent.

18. Use according to Claim 16 of 10, 20, 30, 40 or 50 mg of 2-(3,5-difluorophenyl)-3-(4- methylsulfonyl)phenyl)-2-cyclopenten-1-one in the manufacture of a dosage form of a medicament for the treatment of an inflammatory disease susceptible to treatment with a non-steroidal anti-inflammatory agent.

19. Use according to Claim 16 of 60, 70, 80, 90, 100, 110, or 120 mg of 2-(3,5-difluorophenyl)-3-(4- methylsulfonyl)phenyl)-2-cyclopenten-1-one in the manufacture of a dosage form of a medicament for the treatment of an inflammatory disease susceptible to treatment with a non-steroidal anti-inflammatory agent.

20. Use according to Claim 16 of 10 to 50 mg of 2-(3,5-difluorophenyl)-3-(4- methylsulfonyl)phenyl)-2-cyclopenten-1-one in the manufacture of a dosage form of a medicament for the treatment of an inflammatory disease susceptible to treatment with a non-steroidal anti-inflammatory agent.

21. Use according to Claim 16 of 60 to 120 mg of 2-(3,5-difluorophenyl)-3-(4- methylsulfonyl)phenyl)-2-cyclopenten-1-one in the manufacture of a dosage form of a medicament for the treatment of an inflammatory disease susceptible to treatment with a non-steroidal anti-inflammatory agent.

22. Use according to Claim 16 of 10 to 50 mg of 2-(3,5-difluorophenyl)-3-(4- methylsulfonyl)phenyl)-2-cyclopenten-1-one in the manufacture of a dosage form of a medicament for the treatment of osteoarthritis.

23. Use according to Claim 16 of 60 to 120 mg of 2-(3,5-difluorophenyl)-3-(4- methylsulfonyl)phenyl)-2-cyclopenten-1-one in the manufacture of a dosage form of a medicament for the treatment of osteoarthritis.

24. Use according to Claim 16 of 10 to 50 of 2-(3,5-difluorophenyl)-3-(4- methylsulfonyl)phenyl)-2-cyclopenten-1-one in the manufacture of a dosage form of a medicament for the treatment of rheumatoid arthritis.

25. Use according to Claim 16 of 60 to 120 of 2-(3,5-difluorophenyl)-3-(4- methylsulfonyl)phenyl)-2-cyclopenten-1-one in the manufacture of a dosage form of a medicament for the treatment of rheumatoid arthritis.

26. Use according to Claim 16 of 10 to 50 mg of 2-(3,5-difluorophenyl)-3-(4- methylsulfonyl)phenyl)-2-cyclopenten-1-one in the manufacture of a dosage form of a medicament for the treatment of non-chronic headache, pain or swelling.

27. Use according to Claim 16 of 60 to 120 mg of 2-(3,5-difluorophenyl)-3-(4- methylsulfonyl)phenyl)-2-cyclopenten-1-one in the manufacture of a dosage form of a medicament for the treatment of non-chronic headache, pain or swelling.

28. A compound 2-(3,5-difluorophenyl)-3-(4-methylsulfonyl)phenyl)-2-cyclopenten-1-one.

29. A compound 2-(3,5-difluorophenyl)-3-(4-methylsulfonyl)phenyl)-2-cyclopenten-1-one, for use in therapy.

30. Crystalline 2-(3,5-difluorophenyl)-3-(4-methylsulfonyl)phenyl)-2-cyclopenten-1-one.
